# EUROPEAN PATENT SPECIFICATION

(11) **EP 0 379 367 B1**
(45) Date of publication and mention of the grant of the patent: **01.04.1998**
(21) Application number: 90300521.3
(22) Date of filing: 18.01.1990
(51) Int. Cl.: A61K 7/48, A61K 31/07, A61K 31/19, A61K 31/21

(54) **Method for the prevention of intrinsically aged skin with retinoids**
Verfahren zur Verhütung der Hautalterung mittels Retinoiden
Méthode de prévention par des rétinoides de la peau âgée

(30) Priority: 19.01.1989 US 299119; 20.01.1989 CA 588835
(43) Date of publication of application: 25.07.1990
(60) Divisional application: 97201923.6
(73) Proprietor: ORTHO PHARMACEUTICAL CORPORATION, Raritan New Jersey 08869-0602 (US)
(72) Inventor: Kligman, Albert M., Philadelphia, PA 19103 (US); Mezick, James A., East Brunswick, NJ 08816 (US); Capetola, Robert J., Doylestown, PA 18901 (US)
(74) Representative: Fisher, Adrian John

(56) References cited:
- EP-A- 0 038 246
- EP-A- 0 229 561
- EP-A- 0 253 393
- EP-A- 0 274 104
- WO-A-86/06275
- WO-A-88/07857
- FR-A- 2 612 775
- GB-A- 1 466 062
- US-A- 4 603 146

## Description

### Background of the Invention

### Field of the Invention

The present invention relates to a method for the prevention of intrinsically (chronologically) aged skin by the topical administration of retinoids.

### Description of Prior Art

Cutaneous aging results from two distinct and independent processes: intrinsic aging and photoaging. Although the skin changes that occur in intrinsic aging may involve genetically programmed senescence mediated perhaps by as yet unidentified endogenous factors, an understanding of the clinical alterations as well as changes in the extracellular matrix and molecular biology is beginning to emerge. Clinically, intrinsic aging is characterized by having a thin, atrophic dermis, reduction in subcutaneous adipose tissue and by the presence of fine wrinkling, In addition, there appears an unblemished surface with some deepening of skin surface markings and some histopathologic and ultrastructural alterations as well. Compared to young skin, intrinsically aged skin manifests its most pronounced changes in the dermis. The major changes in the elastic tissue include an increase in the number as well as thickness of fibers, loss of vertical fibers that insert into the basement membrane, and the presence of large irregular fibroblasts containing extensive rough endoplasmic reticulum, characteristic of elastogenesis.

Conversly, photodamaged skin is characterized by coarse wrinkling and furrowing and the skin appears loose, thickened and leathery. Photodamaged skin represents a state of mild chronic inflammation. Histologically, in photodamaged skin, there is excessive accumulation of elastotic material in the upper dermis, basophilic degeneration and homogenization of the collagen fibers accompanying the accumulation of glycosaminoglycan-proteoglycan complexes. Also, there appears to be a loss of collagen bundles. Biochemically, there is a decrease in mature (insoluble) collagen with a concomitant increase in soluble collagen. Mature collagen may be degraded by the chronic inflammatory infiltrate produced by ultraviolet-B irradiation. However, in intrinsically aged skin, mature collagen is more stable and resistant to enzymatic degradation, the bundles become larger forming disoriented rope-like structures. Also, there is marked thickening or acanthosis in the epidermis as well as the dermis and sebaceous glands become greatly enlarged. Electronmicroscopic studies reveal that early changes in photodamaged skin consist of an increase and enlargement of the microfibrillar component of the elastic fibers. In more advanced stages, the elastic fibers are highly disorganized.

Concerning microcirculation, there is a loss of vascular area in both intrinsic and photoaged skin, with distinct differences between the two. Intrinsically-aged skin experiences a uniform reduction in the vasculature with a progressive thinning of the vessel wall. The vessels are not dilated or deranged and have an undisturbed superficial plexus. In photoaged skin, the vasculature is almost totally missing in some areas of the dermis with the horizontal superficial plexus almost completely destroyed, while in other areas the vessels become dilated and tortuous and are seen on the surface of the skin as telangiectasias. In early photodamage, the vessel wall actually thickens and then progressively thins.

Thus, there are distinct differences between intrinsic aging and photoaging. Kligman, A.M. et al., "Cutaneous Aging: The Differences Between Intrinsic Aging and Photoaging", Journal of Cutaneous Aging and Cosmetic Dermatology, Vol. 1, No. 1, pp. 5-12 (1988). These differences are summarized in Table 1.

Tretinoin, all trans retinoic acid, has been described

**TABLE 1**

| Feature | Intrinsic Aging | Photoaging |
|---|---|---|
| Clinical appearance | Smooth, unblemished surface | Nodular, leathery surface with blotches, yellowing |
| | Some deepening of skin surface markings Some loss of elasticity | Deep wrinkles Significant loss of elasticity |
| Epidermis | Thin and viable | Marked acanthosis, cellular atypia |
| Elastic tissue | Increased, but almost normal | Tremendous increase, degenerates into amorphous mass |
| Collagen | Bundles thick, disoriented | Marked decrease of bundles and fibers |
| GAGs, PGs^{a} | Slighty decreased | Markedly increased |
| Reticular dermis | Thinner Fibroblasts decreased, inactive Mast cells decreased, no inflammation | Thickened, elastosis Fibroblasts increased, hyperactive Mast cells markedly increased, mixed inflammatory infiltrate |
| Papillary dermis | No Grenz zone | Solar elastosis with Grenz zone |
| Microvasculature | Moderate loss | Great loss, abnormal and telangiectatic |

| | | |
|---|---|---|
| ^{a}GAGs, glycosaminoglycans; PGs, proteoglycans | | |

as being effective in the treatment of photoaged skin (Weiss, J. J. et al., "Topical Tretinoin Improves Photoaged Skin," Journal of the American Medical Association, Vol. 259, No. 4, pp. 527-532, (1988). Little is known, however, of the effectiveness of retinoids in the treatment or prevention of intrinsically aged skin.

Vitamin-A acid has been suggested as being useful in the treatment of wrinkles, sun damaged and sagging skin (WO 86/06275; Saline, C., "Adventures in the Skin Trade." Philadelphia Magazine, pp. 120-133 (1980)). However, no method is described for the prevention of the symptoms of intrinsically aged skin by the topical administration prior to the appearance of said symptoms of an effective amount of a retinoid in a pharmaceutically acceptable carrier.

The use of retinoids in the treatment of photoaged skin is also disclosed in EP-A-0229561, EP-A-0274104 and GB-A-1466062.

### Summary of the Invention

The present invention is directed to a method for the prevention of the symptoms of intrinsically (chronologically) aged skin by the topical administration prior to the appearance of said symptoms of an effective amount of a retinoid in a pharmaceutically acceptable carrier. The retinoids employed may be any natural and/or synthetic compound which possesses the biological activity of vitamin A.

### Detailed Description of the Invention

The invention relates to the use of retinoids for the prevention of the symptoms of intrinsically (chronologically) aged skin.

Retinoids have been defined narrowly as comprising simply vitamin A (retinol) and its derivatives, such as vitamin A aldehyde (retinal) and vitamin A acid (retinoic acid), which comprise so-called natural retinoids. However, subsequent research has resulted in a much larger class of chemical compounds that are deemed retinoids due to their biological similarity to vitamin A and its derivatives. Compounds useful in the present invention include all natural and/or synthetic analogs of vitamin A or retinol-like compounds which possess the biological activity of vitamin A in the skin. These include: regulation of epithelial cell differentiation of ketatinocytes in the epidermis; stimulation of new collagen synthesis in the dermis, and production of new blood vessels (angiogenesis). Accordingly, as used herein for purposes of the present invention, the term "retinoid" will be understood to include any of the foregoing compounds. Examples of suitable retinoids in the present invention are set forth in Table 2, although it will be understood that the invention is not limited thereto.

**Table 2**

| |
|---|
| all-trans-retinoic acid 13-cis-retinoic acid |
| (all-E)-9-(4-methoxy-2,3,6-trimethylphenyl)-3,7-dimethyl-2,4,6,8-nonatetraenoic acid ethyl ester |
| (all-E)-9-(4-methoxy-2,3,6-trimethylphenyl-3,7-dimethyl-2,4,6,8-nonatetraenoic acid |
| N-ethyl-9-(4-methoxy-2,3,6-trimethylphenyl)-3,7-dimethyl-2,4,6,8-nonatetraenamide |
| (E,E)-9-(2,6-dichloro-4-methoxy-3-methylphenyl)-3,7-dimethyl-2,4,6,8-nonatetraenoic acid ethyl ester |
| 7,8-didehydroretinoic acid |
| (E,E)-4-[2-methyl-4-(2,6,6-trimethyl-1-cyclohexen-1-yl)-1,3-butadienyl]benzoic acid |
| (E)-4-[4-methyl-6-(2,6,6-trimethyl-1-cyclohexen-1-yl)-1,3,5-hexatrienyl]benzoic acid |
| (all-E)-3,7-dimethyl-(3-thienyl)-2,4,6,8-nonatetraenoic acid |
| (E,E,E)-3-methyl-7-(5,6,7,8-tetrahydro-5,5,8,8-tetramethyl-2-naphthalenyl)-2,4,6-octatrienoic acid |
| (E)-6-[2-(2,6,6-trimethyl-1-cyclohexen-1-yl)ethenyl]-2-naphthalenecarboxylic acid |
| (E,E,E)-7-(2,3-dihydro-1,1,3,3-tetramethyl-1H-inden-5-yl)-3-methyl-2,4,6-octatrienoic acid |
| (E)-4-[2-(2,3-dihydro-1,1,3,3-tetramethyl-1H-inden-5-yl)-1-propenyl]benzoic acid |
| (E)-4-[2-(5,6,7,8-tetrahydro-5,5,8,8-tetramethyl-2-naphthalenyl-1-propenyl]benzoic acid |
| (E)-4-[2-(5,6,7,8-tetrahydro-3-methyl-5,5,8,8-tetramethyl-2-naphthalenyl-1-propenyl]benzoic acid |
| (E)-1,2,3,4-tetrahydro-1,1,4,4-tetramethyl-6-(1-methyl-2-phenylethenyl)naphthalene |
| 6-(1,2,3,4-tetrahydro-1,1,4,4-tetramethyl-6-naphthyl)-2-naphthalenecarboxylic acid |
| (E)-6-[2-(4-(ethylsulfonyl)phenyl]-1-methylethenyl]-1,2,3,4-tetrahydro-1,1,4,4-tetramethylnaphthalene |
| 4-[(5,6,7,8-tetrahydro-5,5,8,8-tetramethyl-2-naphthalenyl)ethynyl]benzoic acid |
| (E)-2-(1,1,4,4-tetramethyl-1,2,3,4-tetrahydronaphth-7-yl)-1-[4-tetrazol-5-yl)phenyl]-1-propene |
| (E)-4-[2-(5,6,7,8-tetrahydro-7-hydroxy-5,5,8,8-tetramethyl-2-naphthalenyl)-1-propenyl]benzyl alcohol |

Included among the compounds which can be employed are the pharmaceutically acceptable addition salts and esters of the retinoids such as the palmitates etc. Also encompassed within the term "retinoid" are geometric and stereoisomers of the retinoids.

A pharmacological composition containing a retinoid as the active ingredient in intimate admixture with a pharmaceutical carrier can be prepared according to conventional pharmaceutical compounding techniques, such as, for example, those known for topical application of all-trans-retinoid acid. The carrier may take a wide variety of physical forms such as, for example, creams, dressings, gels, lotions, ointments or liquids. The retinoid will be present in the composition in an amount from about 0.00001% by weight to about 0.2% by weight, depending on the potency of the retinoid. A suitable topical retinoid preparation in a gel vehicle is Retin-A™, which contains 0.01% to 0.1% by weight of the active ingredient, (produced by Ortho Pharmaceutical Corporation).

The following example describes the invention in greater particularity, and is intended to be a way of illustrating but not limiting the invention.

### EXAMPLE 1

Lifetime Application of all-trans-Retinoic Acid to Albino Hairless Mice.

### Materials and Methods

Female Albino hairless mice (Skh-hairless-1), age 8-11 weeks, were treated topically, three times a week, for the remainder of their life span as follows:

| | | |
|---|---|---|
| Group 1. | 0.025% all-trans-retinoic acid (100 µl) | 12 mice |
| Group 2. | Cream vehicle (100 µl) | 12 mice |
| Group 3. | Untreated | 12 mice |

The mice were examined monthly for skin changes and possible weight loss that might indicate a toxic effect.

Toward the end of the study, mice were photographed to show the condition of the skin. A few mice were sacrificed at about 70, 80 and 90 weeks, with dorsal skin taken for histochemical evaluation. The remainder were allowed to live out their life span which ranged from 91-106 weeks and were biopsied when moribound, if possible. The histochemical stains were: H&E for general histology, Luna's for elastic fibers, Van Gieson's for collagen and Mowry's for proteoglycans.

### Results

### A. Toxicity

1. There was no difference in the weights of mice within the three groups.
2. There was no difference in mortality within the three groups.
3. At biopsy and necropsy there was no indication that the mice suffered any adverse or unusual effects.

Therefore: treatment with either all-trans-retinoic acid or vehicle over a lifetime produced no detectable toxic effects.

### B. Clinical Observations

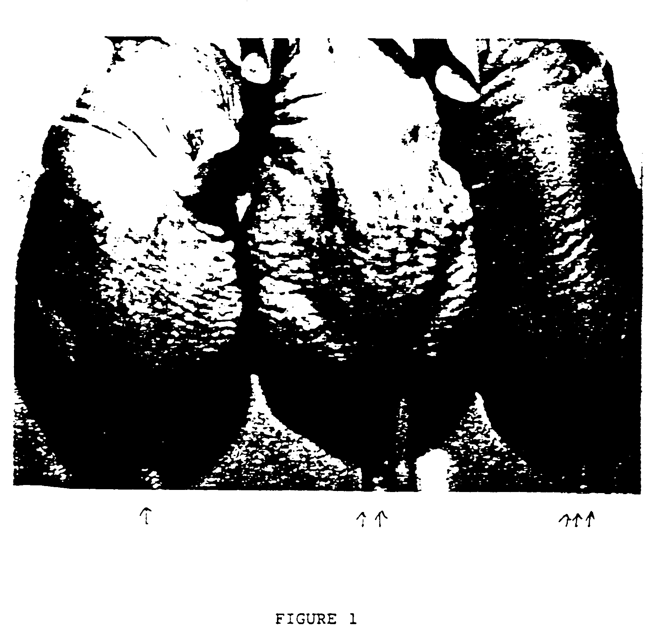

Mice treated with all-trans-retinoic acid had thinner, pinker, more uniform skin than the controls. They more closely resembled far younger, untreated mice. Vehicle controls had thicker, yellower skin. Untreated controls had extremely thick, sagging, yellowed skin, typical of such aged mice (Figure 1).

Figure 1. is a photograph which illustrates the results of lifetime application of vehicle or all-trans-retinoic acid to female albino hairless (Skh-1) mice. ↑ = vehicle, ↑↑ = untreated, ↑↑↑ = all-trans-retinoic acid. Mice were 91-106 weeks of age at photograph. All-trans-retinoic acid mice had thinner, pinker, more uniform skin than controls or untreated mice. They resemble young mice. Vehicle control mice had thicker, yellow skin. Untreated mice had very thick, sagging, yellowed skin.

### C. Histochemical Observations

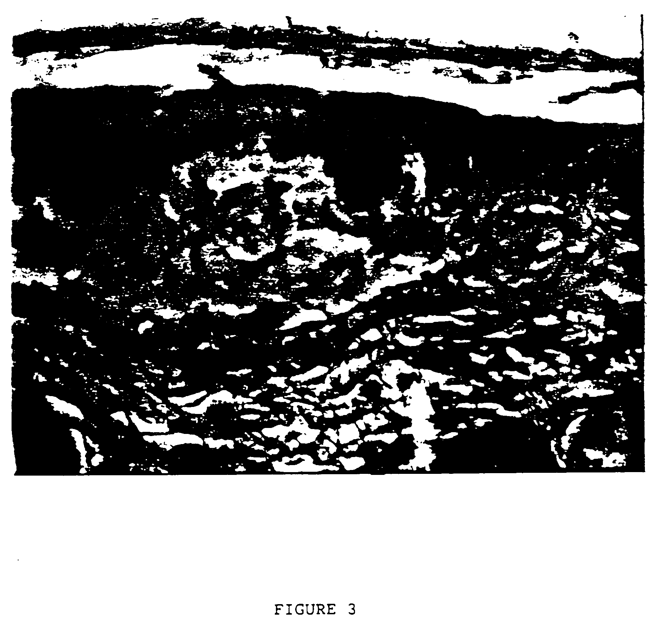

H&E for General Histology (See Figures 2, 3 and 4)

Figure 2. is a picture of a normal old mouse, at 80 weeks of age. H & E stain.

Figure 3 is a picture which illustrates cream vehicle mice at 85 weeks of age.

Figure 4 is a picture which illustrates mice treated with all-trans-retinoic acid. (Lifetime of treatment). Hyperplastic epidermis and strongly granular layer of approximately 4 cells. Cells are plump, cytologically normal and have abundant cytoplasm and an abundance of new blood vessels. Sample was taken from a mouse 95 weeks of age.

In all-trans-retinoic acid-treated specimens, the epidermis was hyperplastic (about 8 cell layers) including a strongly granular layer of about 4 cell layers. The cells were plump, cytologically normal and had an abundant cytoplasm. Control specimens had the usual 2-4 compressed-looking cells. Blood vessels were readily seen in the all-trans-retinoic acid group, unlike in the controls. Areas of new collagen were apparent by the parallel array of the bundles and the presence of numerous large fibroblasts. These regions were free of inflammation despite a mild inflammatory infiltrate in the mid-dermis. No repair areas were seen in the controls and dermal inflammation was present but variable from specimen to specimen.

### Luna's Stain for Elastic Fibers

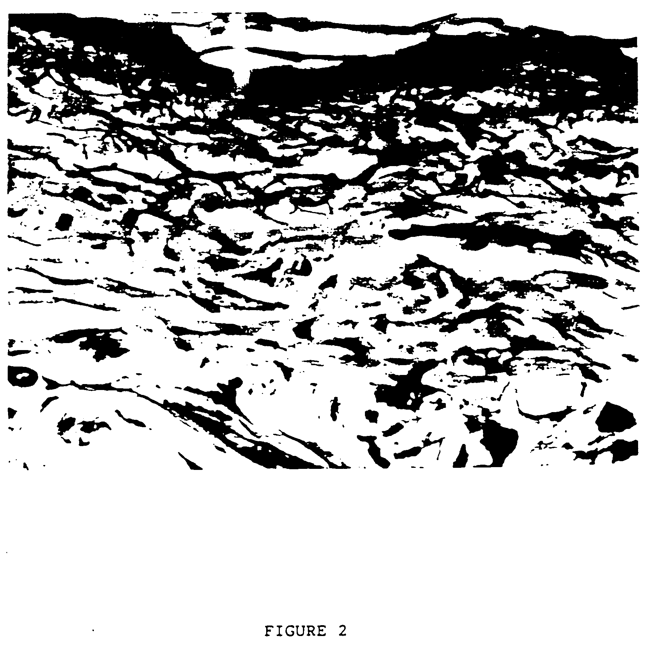

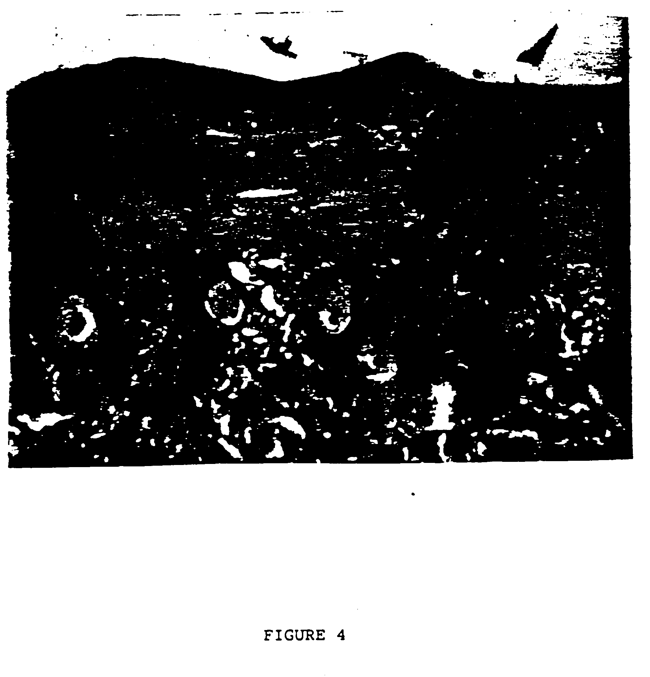

This stain confirmed the presence of new collagen in the sub-epidermal dermis of Retin-A™-treated mice. These areas, termed repair zones, are identified by elastic fibers at their lower border, having been displaced downward by the deposition of new collagen. Repair zones were intermittent across the specimen, unlike in photodamaged skin where they are continuous. In controls, no repair zones were found (Figure 2). In addition, mice treated with all-trans-retinoic acid appeared to have an increased amount of new elastic fibers (Figure 4).

### Van Gieson's Stain for Collagen

Collagen in the repair zones stained mainly as normal, mature collagen.

### Mowry's Stain for Glycosamino- and proteoglycans

There was little or no increase in dermal glycosaminoglycans or proteoglycans in any of the groups except for intermittent deposits at the dermal-epidermal junction. These deposits are typically found in aging mice. Fewer deposits were found in the retinoid treated group.

## Claims

1. A cosmetic method for the prevention of the symptoms of intrinsically aged skin which comprises the topical administration prior to the appearance of said symptoms of an effective amount of a retinoid in a pharmaceutically acceptable carrier.

2. The method of claim 1 wherein the retinoid is all trans retinoic acid.

3. The method of claim 1 wherein the retinoid is 13-cis-retinoic acid.

4. The method of claim 1 wherein the retinoid is retinol.

5. The method of claim 1 wherein the retinoid is selected from the group consisting of:
(E)-4-[4-methyl-6-(2,6,6-trimethyl-1-cyclohexen-1-yl)-1,3,5-hexatrienyl]benzoic acid
(E)-6-[2-(4-(ethylsulfonyl)phenyl]-1-methylethenyl]-1,2,3,4-tetrahydro-1,1,4,4-tetramethylnaphthalene
4-[(5,6,7,8-tetrahydro-5,5,8,8-tetramethyl-2-naphthalenyl)ethynyl]benzoic acid
and (E)-4-[2-(5,6,7,8-tetrahydro-7-hydroxy-5,5,8,8-tetramethyl-2-naphthalenyl)-1-propenyl]benzyl alcohol

6. The method of claim 1 wherein the retinoid is present in an amount from about 0.00001% by weight to 0.2% by weight.

7. The use of a retinoid in the manufacture of a medicament for the therapeutic prevention of the symptoms of intrinsically aged skin, said method comprising administering said retinoid topically in a pharmaceutically acceptable carrier prior to the appearance of said symptoms.

8. The use of a retinoid as claimed in claim 7, wherein said retinoid is as specified in any of claims 2 to 6.

## Patentansprüche

1. Kosmetisches Verfahren zur Verhütung der Symptome von natürlich gealterter Haut, umfassend das topische Verabreichen einer wirksamen Menge eines Retinoids in einem pharmazeutisch verträglichen Träger vor dem Auftreten dieser Symptome.

2. Verfahren nach Anspruch 1, wobei es sich bei dem Retinoid um all-trans-Retinoesäure handelt.

3. Verfahren nach Anspruch 1, wobei es sich bei dem Retinoid um 13-cis-Retinoesäure handelt.

4. Verfahren nach Anspruch 1, wobei es sich bei dem Retinoid um Retinol handelt.

5. Verfahren nach Anspruch 1, wobei das Retinoid aus folgender Gruppe ausgewählt ist:
(E)-4-[4-Methyl-6-(2,6,6-trimethyl-1-cyclohexen-1-yl)-1,3,5-hexatrienyl]-benzoesäure;
(E)-6-[2-(4-(Ethylsulfonyl)-phenyl)-1-methylethenyl]-1,2,3,4-tetrahydro-1,1,4,4-tetramethylnaphthalin;
4-[(5,6,7,8-Tetrahydro-5,5,8,8-tetramethyl-2-naphthalinyl)-ethinyl]-benzoesäure;
und (E)-4-[2-(5,6,7,8-Tetrahydro-7-hydroxy-5,5,8,8-tetramethyl-2-naphthalinyl)-1-propenyl]-benzylalkohol.

6. Verfahren nach Anspruch 1, wobei das Retinoid in einer Menge von etwa 0,00001 bis etwa 0,2 Gew.-% vorhanden ist.

7. Verwendung eines Retinoids bei der Herstellung eines Arzneimittels zur therapeutischen Verhütung der Symptome von natürlich gealterter Haut, wobei das Verfahren das topische Verabreichen des Retinoids in einem pharmazeutisch verträglichen Träger vor dem Auftreten dieser Symptome umfaßt.

8. Verwendung eines Retinoids nach Anspruch 7, wobei es sich beim Retinoid um eines der in den Ansprüchen 2 bis 6 angegebenen Produkte handelt.

## Revendications

1. Méthode cosmétique pour la prévention des symptômes de la peau intrinsèquement vieillie, qui comprend l'administration topique, avant l'apparition desdits symptômes, d'une quantité efficace d'un rétinoïde dans un support pharmaceutiquement acceptable.

2. Méthode de la revendication 1 où le rétinoïde est l'acide tout trans-rétinoïque.

3. Méthode de la revendication 1 où le rétinoïde est l'acide 13-cis-rétinoïque.

4. Méthode de la revendication 1 où le rétinoïde est le rétinol.

5. Méthode de la revendication 1 où le rétinoïde est sélectionné dans le groupe consistant en :
Acide (E)-4-[4-méthyl-6-(2,6,6-triméthyl-1-cyclohexén-1-yl)-1,3,5-hexatriényl]benzoïque
(E)-6-[2-(4-(éthylsulfonyl)phényl]1-méthyléthényl]-1,2,3,4,-tétrahydro-1,1,4,4-tétraméthylnaphtalène
Acide 4-[(5,6,7,8-tétrahydro-5,5,8,8-tétraméthyl-2-naphtalényl)éthynyl]benzoïque
et Alcool (E)-4-[2-(5,6,7,8-tétrahydro-7-hydroxy-5,5,8,8-tétraméthyl-2-naphtalényl)-1-propényl]benzylique

6. Méthode de la revendication 1 où le rétinoïde est présent en une quantité d'environ 0,00001 % en poids à 0,2 % en poids.

7. Utilisation d'un rétinoïde dans la fabrication d'un médicament pour la prévention thérapeutique des symptômes de la peau intrinsèquement vieillie, ladite méthode comprenant l'administration dudit rétinoïde topiquement dans un support pharmaceutiquement acceptable avant l'apparition desdits symptômes.

8. Utilisation d'un rétinoïde selon la revendication 7, où ledit rétinoïde est tel que spécifié selon l'une quelconque des revendications 2 à 6.
